# EUROPEAN PATENT SPECIFICATION

(11) **EP 1 446 131 B1**
(45) Date of publication and mention of the grant of the patent: **09.05.2007**
(21) Application number: 02787601.0
(22) Date of filing: 07.11.2002
(51) Int. Cl.: A61K 31/7024, A61P 9/10

(54) **USE OF SPECIFIC DOSE OF FONDAPARINUX SODIUM FOR THE TREATMENT OF ACS**
DIE VERWENDUNG EINER SPEZIFISCHEN DOSIS VON FONDAPARINUX NATRIUM ZUR BEHANDLUNG DES AKUTEN KORONAREN SYNDROMS
UTILISATION D'UNE DOSE SPECIFIQUE DE FONDAPARINUX SODIQUE POUR TRAITER DES SYNDROMES CORONARIENS AIGUS

(30) Priority: 13.11.2001 EP 01204323
(43) Date of publication of application: 18.08.2004
(73) Proprietor: GLAXO GROUP LIMITED, Greenford, Middlesex UB6 ONN (GB)
(72) Inventor: LENSING, Anthonie Wilhelmus A., c/o N.V. Organon, 5340 BH Oss (NL)
(74) Representative: Sewell, Richard Charles
(86) International application number: PCT/EP2002/012482
(87) International publication number: WO 2003/041722

(56) References cited:
- WO-A-98/56365
- AU-B- 698 456
- DATABASE DRUGNL [Online] R&D Focus Drug News, "Fondaparinux sodium Organon, Sanofi-Synthelabo clinical data" retrieved from STN, accession no. 2001:3794 DRUGNL XP002199103 & "Results from a phase II trial (PENTUA) in patients with acute coronary syndrome, treated with fondaparinux sodium......were presented at the 74th scientific sessions of the American Heart Association, 11-14 November 2001, Anaheim, USA."
- VAN DE WERF, F.: "New data in treatment of acute coronary syndromes" AMERICAN HEART JOURNAL, vol. 142, no. 2 (suppl.), 2001, pages S16-S21, XP001064754
- COUSSEMENT, P.K.: "SR90107A/Org31540, a new synthetic pentasaccharide (SP), as adjunct to fibrinolysis in ST-elevation acute myocardial infarction (AMI): the Pentalyse study." JOURNAL OF AMERICAL COLLEGE OF CARDIOLOGY, vol. 36, no. 1, 2000, pages 317-318, XP002197925
- COUSSEMENT, P.K., ET AL.: "a synthetic factor-Xa inhibitor (Org31540/90107A) as an adjunct to fibrinolysis in acute myocardial infarction. The PENTALYSE study." EUROPEAN HEART JOURNAL, vol. 22, no. 18, 2001, pages 1716-1724, XP001064761 cited in the application
- TURPIE, A.G.G.: "Pentasaccharide Org31540/90107A clinical trials update: lessons for practice" AMERICAN HEART JOURNAL, vol. 142, no. 2 (suppl.), 2001, pages S9-S15, XP001073659
- TOPOL. E.: "Recent advances in anticoagulant therapy for acute coronary syndromes" AMERICAN HEART JOURNAL, vol. 142, no. 2 (suppl.), 2001, pages S22-S29, XP001073658
- HERBERT, J.M., ET AL.: "SR 90107A/Org 31540, a novel anti-factor Xa antithrombotic agent" CARDIOVASCULAR DRUG REVIEWS, vol. 15, no. 1, 1997, pages 1-26, XP001064736
- ERIKSSON, B.I., ET AL.: "Fondaparinux compared with enoxaparin for the prevention of venous thromboembolism after hip-fracture surgery." THE NEW ENGLAND JOURNAL OF MEDICINE, vol. 345, 2001, pages 1298-1304, XP001064757 cited in the application
- BAUER, K.A., ET AL.: "Fondaparinux compared with enoxaparin for the prevention of venous thromboembolism after elective major knee surgery." THE NEW ENGLAND JOURNAL OF MEDICINE, vol. 345, no. 18, 2001, pages 1305-1310, XP001064758 cited in the application
- TURPIE, A.G.G., ET AL.: "A synthetic pentasaccharide for the prevention of deep-vein thrombosis after total hip replacement." THE NEW ENGLAND JOURNAL OF MEDICINE, vol. 344, no. 9, 2001, pages 619-625, XP001064759 cited in the application
- ANONYMOUS: "Dosage and administration:Lovenox" 22 June 2006 (2006-06-22), Retrieved from the Internet: URL:http://www.pdr.net/druginformation/sim plesearchn.aspx>
- ANONYMOUS: "Dosage and administration: Fragmin" 22 June 2006 (2006-06-22), Retrieved from the Internet: URL:http://www.pdr.net/druginformation/sim plesearchn.aspx>
- MAARTE L. SIMOONS, ET. AL.: "A dose-finding study of fondaparinux in patients with Non-ST-segment elevation acute coronary syndromes" JOURNAL OF THE AMERICAN COLLEGE OF CARDIOLOGY, vol. 43, no. 2, 2004, pages 2183-2190,
- TONY SHELDON: "How whistleblowing cost one doctor 550000 pounds" BMJ, vol. 324, 2002, page 1240,

## Description

The invention relates to a new use of a specific dose of fondaparinux sodium for the manufacture of a medicament for the treatment of Acute Coronary Syndromes.

Acute Coronary Syndromes (ACS) represent a major health problem leading to a large number of hospitalizations (1 million in US / 2 to 2.5 million worldwide) (Braunwald E., *et al.* Department of Health and Human Services 1994; 10: 154 / Cannon C.P., Journal of Thrombosis and Thrombolysis 1995; 2: 205-218) and a high mortality/reinfarction rate of 10 % at 3 months to 17 % at 24 months (several studies performed in 1960s and 1970s) (Bertrand M.E., *et al.* European Heart Journal 2000; 21: 1406-1432).

There is pathological and angiographic evidence that ACS result from a ruptured or eroded atherosclerotic plaque with superimposed coronary thrombosis of varying degrees. In patients with non ST-segment elevation ACS (≈ Unstable Angina / non Q-wave Acute Myocardial Infarction (AMI)) the treatment strategy is aimed at alleviating ischaemia and associated symptoms. Antithrombotic drugs are an important component of the therapeutic strategy as recommended by the ACCP consensus conference (Cairns J.A., *et al.* Chest 2001; 119: 228S-252S).

It has been shown that the combination of heparin (UFH) and Acetylsalicylic Acid (ASA) has improved the clinical course of patients with non ST-segment elevation ACS (The RISC group. Lancet 1990; 336: 827-830 / Théroux P., *et al.* Circulation 1993; 88(part 1): 2045-2048 / Cohen M., *et al.* Am J Cardiol 1994; 89: 81-88 / Oler A., *et al.* JAMA 1996; 276: 811-815). A meta-analysis by Eikelboom J.W., *et al.* (Lancet 2000; 355: 1936-1942) suggests that the addition of UFH or LMWH (Low-Molecular-Weight-Heparins) to ASA up to 7 days reduces the incidence of non-fatal MI or death by about 50 % in patients with non ST-segment elevation ACS. A meta-analysis of two individual trials using enoxaparin suggests superiority of enoxaparin over UFH (Antman E.M., *et al.* Circulation 1999; 100: 1602-1608). Currently enoxaparin is the most widespread used LWMH in the acute phase treatment of non ST-segment elevation ACS.

Both UFH and LMWH have an effect on several stages of the blood coagulation cascade, both inhibiting factor Xa and thrombin (factor IIa). Factor Xa catalyzes the generation of thrombin and subsequently thrombin regulates the last step in the coagulation cascade. The prime function of thrombin is the cleavage of fibrinogen to generate fibrin monomers, which form an insoluble gel by cross-linking, thereby initiating thrombus formation.
As a new antithrombotic product, fondaparinux sodium (described in Chemical Synthesis to Glycosaminoglycans, Supplement to Nature 1991, *350,* 30-33), which is a pure factor Xa inhibitor, retains advantages of the LMWHs, like subcutaneous administration and no biological monitoring, and has additional advantages, like a controlled total chemical synthesis. It has been demonstrated in early clinical settings, that fondaparinux sodium is effective in ACS. (Pentalyse study, Eur Heart J, 2001; 22: 1716-1724).

For treating patients with ACS the dose of choice of UFH or LMWHs is always a three to four times higher dose than the dose required for the prophylaxis of deep vein thrombosis (DVT) (see e.g. Turpie A.G.G., *et al.,* Arch Intern Med / Vol. 161, June 25, 2001 and references cited). Since the dose of fondaparinux sodium for prophylaxis of DVT is 2.5 mg (Turpie A.G.G., *et al.* N Engl J Med 2001; 344: 619-25 / Eriksson B.I., *et al.* N Engl J Med 2001; 345: 1298-1304 / Bauer K.A., *et al.* N Engl J Med 2001; 345: 1305-10), the dose for the treatment of ACS would, in line with common practice, be about 7.5-10 mg daily. As a matter of facts, 4, 6, 8, 10 or 12 mg of fondaparinux sodium was tested for the treatment of acute myocardial infarction with ST-segment elevation (Coussement, P.K. et. al., European Heart Journal 2001, Vol. 22 (18), page 1716-1724).

Surprisingly an contrary to common practice in the art, it has now been found that a dose of as low as 2.5 mg of the pentasaccharide methyl O-(2-deoxy-2-sulfoamino-6-O-sulfo-a-D-glucopyranosyl)-(1→4)-O-(β-D-glucopyranosyl uronic acid)-(1→4)-O-(2-deoxy-2-sulfoamino-3,6-di-O-sulfo-α-D-glucopyranosyl)-(1→4)-O-(2-O-sulfo-α-L-idopyranosyl uronic acid)-(1→4)-2-deoxy-2-sulfoamino-6-O-sulfo-α-D-glucopyranoside or a pharmaceutically acceptable salt thereof (in particular its decasodium salt fondaparinux sodium) is effective and safe for treating patients with of ACS.

A pharmaceutically acceptable salt herein is: a salt with counter-ions like alkali or earth-alkali metal ions, like sodium, calcium, or magnesium.

Since therapeutic regimens for ACS consist of a combination of antithrombotic and (an increasing array of) antiplatelet therapies, which, together with invasive procedures, might lead to an increased bleeding risk, the lowest dose of an anticoagulant which is effective and safe is the most preferred dose.
The dose of this invention is in particular preferred for the treatment of non-ST-elevation ACS (comprising unstable angina and non-Q-wave myocardial infarction).

The dose of the pentasaccharide of this invention is administered as a subcutaneous injection to the patient undergoing treatment. Preferably, the patient is a human.

The pentasaccharide may be used as a pharmaceutical composition comprising said pentasaccharide together with pharmaceutically acceptable auxiliaries and optionally other therapeutic agents. The term "acceptable" means being compatible with the other ingredients of the composition and not deleterious to the recipients thereof
The pharmaceutical composition for parenteral administration of the dose of the pentasaccharide of this invention may be presented in unit-dose or multi-dose containers, e.g. injection liquids in predetermined amounts, for example in sealed vials and ampoules, and may also be stored in a freeze dried (lyophilized) condition requiring only the addition of sterile liquid carrier, e.g. water, prior to use.
Mixed with such pharmaceutically acceptable auxiliaries and liquids, e.g. as described in the standard reference, Gennaro et al., Remington's Pharmaceutical Sciences, (18th ed., Mack Publishing Company, 1990, see especially Part 8: Pharmaceutical Preparations and Their Manufacture), the pentasaccharide can be applied as a fluid composition, an injection preparation, in the form of a solution, suspension or emulsion. Aqueous suspensions, isotone saline solutions and sterile injectable solutions may be used, containing pharmaceutically acceptable dispersing agents and/or wetting agents, such as propylene glycol or butylene glycol. The preferred pharmaceutical composition is an isotone saline solution of the pentasaccharide.
The pharmaceutical composition according to the invention may also be presented in the form of a veterinary composition, such compositions may be prepared by methods conventional in the art.

The invention is further illustrated by the following example.

### EXAMPLE

### Dose ranging study of fondaparinux sodium in patients with unstable angina pectoris

A double blind, randomized, controlled dose ranging study, was performed testing in parallel four fondaparinux sodium daily dose levels (2.5, 4.0, 8.0 and 12.0 mg) and a dose regimen of enoxaparin (1 mg/Kg bid = twice daily). Efficacy (composite parameter of death, AMI, recurrent ischemia) and safety of the respective doses of fondaparinux sodium were assessed in patients with unstable angina / non Q-wave MI. Further, safety and efficacy of the four different dose levels of fondaparinux sodium and enoxaparin 1 mg/Kg bid were compared.

### 1. Efficacy Assessment

The primary efficacy endpoint consisted of the composite of the following ischemic events starting from first active drug administration up to and including Day 9 (with Day 1 being the day of first active drug administration):
- Death from any cause except for death related to bleeding
- Acute myocardial infarction (AMI), according to the definition in the protocol
- Symptomatic recurrent ischemia (excluding episodes of ischemia during or at any time after CABG (Coronary Artery Bypass Graft) or PTCA (Percutaneous Transluminal Coronary Angioplasty)) or any ischemia on the 48-h continuous 12-lead ECG monitoring (Mortara).

In the event a patient experienced one of these events, the binary composite outcome was regarded as a treatment failure. In contrast, if none of the above events applied, the composite ischemic outcome was considered as a treatment success.

### Efficacy Analyses

The primary analysis for efficacy parameters was performed on the per protocol (PP) population. The choice of the patients to be excluded from PP population for efficacy analyses was defined before unblinding the trial.

### Primary Efficacy Analysis

The results of the primary efficacy analysis are provided in Table 1.

**Table 1. Primary Efficacy Evaluation according to protocol definition of Myocardial Infarction from first active injection to Day 9 - Corresponding Per Protocol Group excluding patients with insufficient Mortara data and no endpoint until Day 9**

| | | **Fondaparinux sodium** | | | | | **Enoxaparin** |
|---|---|---|---|---|---|---|---|
| | | **2.5 mg** | **4 mg** | **8 mg** | **12 mg** | **All** | |
| | | **(N=203)** | **(N=177)** | **(N=173)** | **(N=187)** | **(N=740)** | **(N=189)** |
| Composite endpoint | n (%) | 61 (30.0%) | 77 (43.5%) | 71 (41.0%) | 65 (34.8 %) | 274 (37.0%) | 76 (40.2%) |
| Death (not related to bleeding) | n (%) | 2(1.0%) | 5(2.8%) | 1(0.6%) | 1(0.5%) | 9(1.2%) | 1(0.5%) |
| AMI n (%) | | 1 (0.5%) | 3 (1.7%) | 5 (2.9%) | 4 (2.1%) | 13 (1.8%) | 3 (1.6%) |
| Symptomatic recurrent ischemia | n (%) | 26 (12.8%) | 33 (18.6%) | 29 (16.8%) | 28(15.0%) | 116 (15.7%) | 36 (19.0%) |
| Recurrent ischemia (Mortara data) | n (%) | 47 (23.2%) | 63 (35.6%) | 55 (31.8%) | 56 (29.9%) | 221 (29.9%) | 59 (31.2%) |

No dose-response for the primary endpoint was observed for the four fondaparinux sodium groups.

### Secondary Efficacy Analysis

Analyses of the secondary endpoints are summarized in Table 2.

**Table 2.Clinical endpoint according to protocol definition of Myocardial Infarction from first active injection to Day 9 - Corresponding Per Protocol Group including patients with insufficient Mortara data**

| | | **Fondaparinux sodium** | | | | **Enoxaparin** |
|---|---|---|---|---|---|---|
| | | **2.5 mg** | **4 mg** | **8 mg** | **12 mg** | |
| | | **(N=210)** | **(N=185)** | **(N=183)** | **(N=199)** | **(N=206)** |
| Death (not related to | n (%) | 2 (1.0%) | 5 (2.7%) | 1 (0.5%) | 1 (0.5%) | 1 (0.5%) |
| bleeding) | | | | | | |
| AMI | n(%) | 1(0.5%) | 3(1.6%) | 5(2.7%) | 4(2.0%) | 3 (1.5%) |
| Recurrent Ischemia | n(%) | 26(12.4%) | 33 (17.8%) | 29(15.8%) | 28 (14.1%) | 36 (17.5%) |
| (Symptomatic) | | | | | | |
| Death or AMI | n (%) | 3 (1.4%) | 8 (4.3%) | 6 (3.3%) | 5(2.5%) | 4 (1.9%) |
| Death or AMI or | n (%) | 27 (12.9%) | 39 (21.1%) | 33 (18.0%) | 30 (15.1%) | 38 (18.4%) |
| recurrent ischemia (Symptomatic) | | | | | | |

### 2. Safety Assessment

The primary safety endpoint was the incidence of major bleeding from first active drug administration up to and including Day 9 as adjudicated by a blinded Central Adjudication Committee (CAC). The incidence of any major or minor bleeding was considered as a secondary safety endpoint.
Other safety endpoints include deaths and all adverse events (latter not reported here).
The safety analysis was performed on the 'all treated patients' (ATP) population.

### Safety Analysis - Bleedings

The incidences of major, minor and any bleedings are provided in Table 3.

**Table 3. Number (%) of patients with adjudicated bleeding events from first active injection to Day 9. All treated patients group**

| | | **Fondaparinux sodium** | | | | **Enoxaparin** |
|---|---|---|---|---|---|---|
| | | **2.5 mg** | **4 mg** | **8 mg** | **12 mg** | |
| | | **(N=229)** | **(N=222)** | **(N=223)** | **(N=238)** | **(N=231)** |
| Major bleeding event | n (%) | 0 (0.0%) | 3 (1.4%) | 4 (1.8%) | 1 (0.4%) | 0 (0.0%) |
| Minor bleeding event | n (%) | 9 (3.9%) | 9 (4.1%) | 9 (4.0%) | 10(4.2%) | 11 (4.8%) |
| Any bleeding event | n (%) | 9 (3.9%) | 12 (5.4%) | 12 (5.4%) | 11 (4.6%) | 11 (4.8%) |

No relevant differences between treatment groups were observed for either major, minor or 'any bleeding' (ie.e major and/or minor).
No major bleedings were observed in the 2.5 mg fondaparinux sodium and enoxaparin groups.
Bleeding risk in the ACS treatment population is heterogenous and related to the incidence of revascularization (PTCA and/or CABG). In the present study the incidence of major bleeding was low and most events were related to coronary intervention. From the 8 major bleedings in this study, 3 events were related to CABG and 3 events were related to coronary angiography. One major bleeding (4 mg Fondaparinux sodium group) occurred after thrombolytic therapy. Therefore, the only major bleeding with no mitigating factors is the one observed in the 12 mg Fondaparinux sodium group. This type of major bleeding (abdominal hematoma) has been reported in other trials assessing the efficacy and the safety of low molecular weight heparin, in the treatment of patients with DVT (Levine M., *et al.* N Engl J Med 1996; 334: 677-81).

### Safety Analysis - Deaths

The incidences of deaths are summarized in Table 4.

**Table 4. Number (%) of patients who died from first active injection onwards - All treated patients group**

| | **Fondaparinux sodium** | | | | **Enoxaparin** |
|---|---|---|---|---|---|
| | **2.5 mg** | **4 mg** | **8 mg** | **12 mg** | |
| | **(N=229)** | **(N=222)** | **(N=223)** | **(N=238)** | **(N=231)** |
| Patients with SAE from First injection | | | | | |
| - Leading to death between first injection and day 30¹ | 3 (1.3%) | 7 (3.2%) | 4 (1.8%) | 6 (2.5%) | 3 (1.3%) |
| - Leading to death after day 30 | 1 (0.4%) | 3 (1.4%) | 2 (0.9%) | 1 (0.4%) | 3 (1.3%) |
| Total deaths reported | 4 (1.7%) | 10 (4.5%) | 6 (2.7%) | 7 (2.9%) | 6 (2.6%) |

| | | | | | |
|---|---|---|---|---|---|
| ¹Deaths occurring up to Day 30 are also included in the Primary/secondary efficacy endpoints | | | | | |

No relevant differences in the percentage of deaths between treatment groups was observed. Most deaths until day 30 were caused by a cardiac event as judged by the Central Adjudication Committee.

### Summary and Conclusions

- All fondaparinux sodium doses appeared to be efficacious and at least as effective as enoxaparin 1mg/Kg bid with an incidence of the composite endpoint of 37.0% in all fondaparinux sodium groups pooled compared to 40.2% in the enoxaparin group.
- No significant differences between fondaparinux sodium groups and the enoxaparin group were shown for safety parameters.
- Based on the efficacy and safety data, 2.5 mg fondaparinux sodium is the optimum dose for the treatment of ACS. Lowest effective and safe dose. (As shown in Table 2, fewer patients tended to experience death or AMI in the 2.5 mg fondaparinux sodium group compared with the other fondaparinux sodium groups).

## Claims

1. A use of a dose of 2.5 mg of the pentasaccharide methyl O-(2-deoxy-2-sulfoamino-6-O-sulfo-α-D-glucopyranosyl)-(1→4)-O-(β-D-glucopyranosyl uronic acid)-(1→4)-O-(2-deoxy-2-sulfoamino-3,6-di-O-sulfo-α-D-glucopyranosyl)-(1→4)-O-(2-O-sulfo-α-L-idopyranosyl uronic acid)-(1→4)-2-deoxy-2-sulfoamino-6-O-sulpho-α-D-glucopyranoside or a pharmaceutically acceptable salt thereof for the preparation of a medicament for the treatment of Acute Coronary Syndromes (ACS).

2. The use of claim 1, wherein the pentasaccharide is in the form of its decasodium salt.

3. The use of claim 1 or 2 for the treatment of non-ST-elevation ACS.

## Patentansprüche

1. Verwendung einer 2,5 mg-Dosis des Pentasaccharids Methyl-O-(2-desoxy-2-sulfoamino-6-O-sulfo-α-D-glucopyranosyl)-(1→4)-O-(β-D-glucopyranosyluronsäure)-(1→4)-O-(2-desoxy-2-sulfoamino-3,6-di-O-sulfo-α-D-glucopyranosyl)-(1→4)-O-(2-O-sulfo-α-L-idopyranosyluronsäure)-(1→4)-2-desoxy-2-sulfoamino-6-O-sulfo-α-D-glucopyranosid oder eines pharmazeutisch verträglichen Salzes davon für die Herstellung eines Arzneimittels zur Behandlung des akuten Koronarsyndroms (ACS).

2. Verwendung nach Anspruch 1, wobei das Pentasaccharid in Form seines Decanatriumsalzes vorliegt.

3. Verwendung nach Anspruch 1 oder 2 zur Behandlung von Nicht-ST-Hebungs-ACS.

## Revendications

1. Utilisation d'une dose de 2,5 mg du pentasaccharide méthyl-O-(2-désoxy-2-sulfoamino-6-O-sulfo-α-D-glucopyranosyl)-(1→4) -O- (β-D-glucopyranosyl-acide uronique)-(1→4) -O-(2-désoxy-2-sulfoamino-3,6-di-O-sulfo-α-D-glucopyranosyl)-(1→4)-O-(2-O-sulfo-α-L-idopyranosyl-acide uronique)-(1→4)-2-désoxy-2-sulfoamino-6-O-sulfo-α-D-glucopyranoside ou d'un de ses sels pharmaceutiquement acceptables pour la préparation d'un médicament destiné au traitement de syndromes coronariens aigus (ACS).

2. Utilisation suivant la revendication 1, dans laquelle le pentasaccharide est sous forme de son sel décasodique.

3. Utilisation suivant la revendication 1 ou 2 pour le traitement de ACS sans élévation du segment ST.
